# EUROPEAN PATENT SPECIFICATION

(11) **EP 2 431 046 B1**
(45) Date of publication and mention of the grant of the patent: **18.02.2015**
(21) Application number: 10774593.7
(22) Date of filing: 14.05.2010
(51) Int. Cl.: A61K 45/06, A61K 35/28

(54) **USE OF OLIVE LEAF EXTRACTS IN A PHARMACEUTICAL COMPOSITION FOR INDUCING ANGIOGENESIS AND VASCULOGENESIS**
VERWENDUNG VON OLIVENBLATTEXTRAKTEN IN EINER PHARMAZEUTISCHEN ZUSAMMENSETZUNG ZUR INDUKTION VON ANGIOGENESE UND VASKULOGENESE
UTILISATION D'EXTRAITS DE FEUILLES D'OLIVIER DANS UNE COMPOSITION PHARMACEUTIQUE EN VUE D'INDUIRE L'ANGIOGENÈSE ET LA VASCULOGENÈSE

(30) Priority: 14.05.2009 ES 200901296
(43) Date of publication of application: 21.03.2012
(73) Proprietor: Sanidad Y Residencias 21, S.A., 14006 Córdoba (ES); Servicio Andaluz de Salud, 41001 Sevilla (ES); QUESPER R&D, S.L., 14004 Córdoba (ES)
(72) Inventor: QUESADA GÓMEZ, José Manuel, E-14006 Córdoba (ES); SANTIAGO MORA, Raquel María, E-14006 Córdoba (ES); CASADO DÍAZ, Antonio, E-14006 Córdoba (ES); LUQUE DE CASTRO, María Dolores, E-14006 Córdoba (ES)
(74) Representative: Carlos Hernando, Borja
(86) International application number: PCT/ES2010/070327
(87) International publication number: WO 2010/130869

(56) References cited:
- EP-A1- 1 481 669
- WO-A1-2010/070183
- DE-U1-202005 007 222
- ES-A1- 2 274 721
- JP-A- 2008 081 425
- JP-A- 2008 273 938
- US-A1- 2002 110 600
- US-A1- 2003 004 117
- Natural remedies: "Oleuropein", , 12 May 2010 (2010-05-12), XP002698917, Retrieved from the Internet: URL:http://www.naturalremedies.org/oleurop ein/ [retrieved on 2013-06-17]
- KOCA UFUK ET AL: "Wound repair potential of Olea europaea L. leaf extracts revealed by in vivo experimental models and comparative evaluation of the extracts' antioxidant activity.", January 2011 (2011-01), JOURNAL OF MEDICINAL FOOD 2011 JAN-FEB, VOL. 14, NR. 1-2, PAGE(S) 140 - 146, XP002698918, ISSN: 1557-7600 * the whole document *
- ALTINYAY, C. ET AL.: 'HPLC analysis of oleuropein in Olea europaea L.' ANKARA ECZ. FAK. DERG. vol. 35, no. 1, 2006, pages 1 - 11, XP008154468
- GOULAS, V. ET AL.: 'Phytochemicals in olive-leaf extracts and their antiproliferative activity against cancer and endothelial cells' MOLECULAR NUTRITION & FOOD RESEARCH vol. 53, no. 5, 01 May 2009, pages 600 - 608, XP008154470
- BRAUN, L. ET AL.: 'Olive-leaf extract' JOURNAL OF COMPLEMENTARY MEDICINE vol. 4, no. 3, 2005, pages 69 - 73, XP008164684
- MICOL, V. ET AL.: 'New applications of herbal extracts for functional food and pharmaceuticals. Part 2' AGRO FOOD INDUSTRY HI-TECH vol. 14, no. 6, 2003, pages 14 - 16, XP001538408
- KHAYYAL, M. T. ET AL.: 'Blood pressure lowering effect of an olive leaf extract (Olea europaea) in L-NAME induced hypertension in rats' ARZNEIMITTEL-FORSCHUNG vol. 52, no. 11, 2002, pages 797 - 802, XP001526063
- LEPORATTI MARIA LUCIA ET AL: "Comparative analysis of medicinal plants used in traditional medicine in Italy and Tunisia", JOURNAL OF ETHNOBIOLOGY AND ETHNOMEDICINE, BIOMED CENTRAL, LONDON, GB, vol. 5, no. 1, 26 October 2009 (2009-10-26), page 31, XP021064470, ISSN: 1746-4269, DOI: 10.1186/1746-4269-5-31
- FEHRI B ET AL: "Olea europaea L.: Stimulant, anti-ulcer and anti-inflammatory effects", BOLLETTINO CHIMICO FARMACEUTICO, SOCIETA EDITORIALE FARMACEUTICA, MILANO, IT, vol. 135, no. 1, 1 January 1996 (1996-01-01), pages 42-49, XP009131576, ISSN: 0006-6648
- FEHRI B ET AL: "Olea europaea L.: Stimulant, anti-ulcer and anti-inflammatory effects", BOLLETTINO CHIMICO FARMACEUTICO, SOCIETA EDITORIALE FARMACEUTICA, MILANO, IT, vol. 135, no. 1, 1 January 1996 (1996-01-01), pages 42-49, XP009131576, ISSN: 0006-6648

## Description

### Field and Object of the Invention

The present invention is comprised in the medical-pharmaceutical sector and specifically in the technical field of promoting angiogenesis and vascularization, endothelial repair and the treatment of wounds and ulcers.

The object of the present invention is the use of olive leaf extracts for producing a pharmaceutical composition capable of inducing angiogenesis and vascularization. In general, this composition can be used for therapeutic applications in order to promote the differentiation of stem cells into endothelial progenitor cells (EPCs) and/or mature endothelial cells, as well as to induce vessel formation from said endothelial cells. The vascularization-inducing angiogenic composition of the invention is intended for, *inter alia,* cardiovascular disease, ischemic processes in general, ulcers, and wound healing in both human and veterinary medicine.

### State of the Art

Blood vessel formation and remodeling occurs in processes referred to as vasculogenesis, angiogenesis and arteriogenesis, Carmeliet, P. (2004). "Manipulating angiogenesis in medicine." J Intern Med 255(5): 538-61. Vasculogenesis consists of new blood vessel formation from endothelial precursor cells (EPCs) migrating and differentiating into endothelial cells in those sites where vascularization begins. Angiogenesis consists of new capillary branch formation from existing blood vessels. In turn, arteriogenesis relates to the remodeling of an existing artery as a consequence of its adaptation to the blood flow.

Arteriogenesis differs from angiogenesis in its mechanism. The former primarily occurs as a consequence of physical stress (e.g. the rupture or obstruction of a vessel) and the latter primarily occurs due to hypoxia.

Physiologically speaking, the organism regulates angiogenesis through a series of mechanisms acting like on and off switches. The on switches are known as angiogenic growth factors, whereas the "off' switches are known as endogenous angiogenic inhibitors. When there is an uncontrolled and/or excessive increase of angiogenesis, pathologies such as cancer, atherosclerosis and diabetic retinopathy, *inter alia,* can develop. Meanwhile, a reduction of angiogenesis favors the development of cardiovascular diseases, ischemic processes in general, ulcers and difficulty in the wound healing, etc. The treatment of pathologies associated with minor angiogenesis comprises the use of growth factors which promote the vessel formation, such as the endothelial growth factor (EGF), vascular endothelial growth factor (VEGF), fibroblast growth factor (FGF), hypoxia-inducible factor-1 alpha (HIF-1α), FGF-4, hepatocyte growth factor (HGF), tissue kallikrein-related peptidase (TK), proteinase-activated receptor activators (PAR-activators), thrombin, frizzled-A protein and nitric acid [Madeddu, P. (2005). "Therapeutic angiogenesis and vasculogenesis for tissue regeneration." Exp Physiol 90(3): 315-26], *inter alia.*

The use of growth factors such as VEGF for the treatment of ischemic processes is still not well-established because it is difficult to apply the right dosage and because it can cause significant side effects such as hypotension and tissue edemas. [Simons M. and Ware J.A. (2003) "Therapeutic angiogenesis in cardiovascular disease." Nat Rev Drug Discov 2(11):863-71].

Cell therapies applying stem cells or EPCs for promoting angiogenesis for therapeutic purposes are currently being tested as well [Tarzami, S. T. and J. P. Singh (2004) "Pharmacological revascularisation in coronary and peripheral vascular disease." Expert Opin Investig Drugs 13(10): 1319-26)]. Due to their plasticity, stem cells can differentiate into vascular and non-vascular elements, so they can regenerate the part damaged by ischemia. Thus, for example, in the case of myocardial infarction, said cells can further differentiate into myocytes, recovering the damaged tissue.

The strategy of using cell therapy has the drawback of the loss of EPC functionality a few hours after the transplant. To reduce this phenomenon, the following has been proposed: 1) a local rather than systemic release of cells, 2) administration of chemokines which promote EPC mobilization, 3) enrichment and expansion of the EPC cultures and 4) increase of EPC functionality due to genetic modification [Madeddu, P. (2005). "Therapeutic angiogenesis and vasculogenesis for tissue regeneration." Exp Physiol 90(3): 315-26].

The new vessel formation is also involved in wound healing, where different stages are distinguished: angiogenesis, collagen deposit, granulation tissue formation, wound epithelization and contraction. EPCs are involved in angiogenic processes occurring during healing [Eming, S.A., Smola, H. Krieg, T. Treatment of chronic wounds: state of the art and future concepts. Cells Tissues Organs 2002; 172, 105-17].

In later phases of the wound healing, additional angiogenesis is induced by the increase of VEGF in the damaged tissue, with a maximum being several days after the damage [Karayiannakis AJ, Zbar A, Polychronidis A, Simopoulos C. Serum and drainage fluid vascular endothelial growth factor levels in early surgical wounds. Eur Surg Res 2003; 35, 492-6], therefore angiogenesis and related factors are fundamental in the wound healing and repair process. Likewise, the presence of VEGF at specific concentrations of mesenchymal stem cells (MSCs) in the culture medium is the cause of the differentiation of these cells into endothelial cells [Liu, JW, Dunoyer-Geindre S, Serre-Beinier V, Mai G, Lambert JF, Fish RJ, Pernod G, Buehler L, Bounameaux H, Kruithof EK. Characterization of endothelial-like cells derived from human mesenchymal stem cells. J Thromb Haemost 2007; 5: 826-34].

The vascular system in tumors develops in a disorderly and irregular manner. As a consequence, the blood supply in the tumor is inadequate and causes a reduction of chemo- and radiotherapy efficacy [Jain, R. K. (2005). "Normalization of tumor vasculature: an emerging concept in antiangiogenic therapy." Science 307(5706): 58-62]. Therefore, the most innovative therapies today against cancer are using the combined action of anti- and proangiogenic agents for reverting the abnormality of the vascular system in the tumor [Ellis, L. M. and D. J. Hicklin (2008). "VEGF-targeted therapy: mechanisms of anti-tumour activity." Nat Rev Cancer 8(8): 579-91]. Today, a considerable number of plant extracts rich in antioxidant compounds are being studied not only for their antioxidant activity but also for their effect on different physiological processes such as inflammation, cell differentiation, tumorigenesis or angiogenesis, for example patent WO 2005/105127 describes the use of a citrus fruit skin extract capable of accelerating wound healing in rats and promoting angiogenesis.

### Description of the Invention

The present invention describes the use of olive leaf extracts for being used alone, in combination with other compounds or with stem cells for the treatment of pathologies in which the induction of angiogenesis and vasculogenesis is necessary.

An object of the present invention is a pharmaceutical composition containing olive leaf extracts which comply with the definition of olive leaf extract published in Pharmeuropa Olive [(2007) Leaf dry extract Pharmeuropa 19(3): 510-511] for use in the induction of angiogenesis and vasculogenesis.

In a first aspect, the present invention relates to the use of olive leaf extracts for preparing a pharmaceutical composition.

In another aspect, the present invention relates to the use of olive leaf extracts for preparing a pharmaceutical composition for inducing angiogenesis and vasculogenesis and related biological processes such as healing, endothelial repair, ischemic processes, etc.

In a preferred embodiment, the pharmaceutical composition used is presented in the form of a cream, ointment, balsam, solution, emulsion or salve for topical administration.

In another preferred embodiment, the pharmaceutical composition comprises a concentration of less than 30% of olive leaf extract.

In another preferred embodiment, the pharmaceutical composition comprises between 10⁻¹ and 10⁻¹⁰ M of oleuropein contained in the olive leaf extract.

In another preferred embodiment, the pharmaceutical composition is applied between 1 and 8 times a day.

In another preferred embodiment, the pharmaceutical composition is applied together with another product.

In another preferred embodiment, the pharmaceutical composition is applied for the first time up to 14 days after the onset of the damage.

In another preferred embodiment, the pharmaceutical composition is applied by means of a transdermal patch.

In another aspect, the present invention relates to a pharmaceutical composition comprising polyphenol and/or angiogenesis- and vasculogenesis-stimulating factor enriched olive leaf extracts.

In a preferred embodiment, the pharmaceutical composition further comprises mesenchymal stem cells.

In another preferred embodiment, the pharmaceutical composition is presented in the form of a cream, ointment, balsam, solution, emulsion or salve for topical administration.

In another preferred embodiment, the pharmaceutical composition comprises a concentration of less than 30% of olive leaf extract.

In another preferred embodiment, the pharmaceutical composition is applied between 1 and 8 times a day.

In another preferred embodiment, the pharmaceutical composition is applied together with another product.

In another preferred embodiment, the pharmaceutical composition is applied by means of a transdermal patch.

In another preferred embodiment, the pharmaceutical composition is applied for the first time up to 14 days after the onset of the damage.

In a final aspect, the present invention relates to a method for the treatment and/or prevention of wounds or ulcers comprising the application of a pharmaceutical composition as described above.

Specifically, the composition containing olive leaf extracts can be used, alone or associated with other substances, in the formation of mesenchymal stem cell-derived endothelial cells and also in vessel formation from endothelial cells.

Another object of the present invention is the use of olive leaf extracts in the preparation of a pharmaceutical composition for inducing angiogenesis and vasculogenesis.

Vascular endothelial growth factor (VEGF) is a signaling protein involved in vasculogenesis (de novo formation of the circulatory system) and in angiogenesis (growth of blood vessels from preexisting vessels). Actions of VEGF have been studied in vascular endothelial cells, although it also has effects on other cell types (for example, it stimulates the monocyte/macrophage, neuron, renal epithelial cell and tumor cell migration). It has been demonstrated *in vitro* that VEGF stimulates endothelial cell division and migration. VEGF is also a vasodilator and increases vascular permeability; it was originally called vascular permeability factor.

The olive leaf extract used in the present invention is capable of stimulating VEGF synthesis and therefore vasculogenesis and angiogenesis. These processes are the first step for tissue recovery, so olive leaf extract is capable of promoting healing in wounds and ulcers, particularly those occurring in diabetic and/or elderly patients.

The composition is prepared for oral, rectal, parenteral, intraperitoneal, interdermal, transdermal, topical, intratracheal, intramuscular, intravenous administration or for inhalation.

As usually occurs in the preparation of pharmaceutical formulations, in the formulation of the present invention, in addition to the active ingredient oleuropein and/or olive leaf extract, all those ingredients which are conventional for a person skilled in galenic practice which allow the best dosage, storage, penetration and bioavailability properties of said active ingredients, so that they can act with the highest possible efficacy will be incorporated at different concentrations. By way of example, stabilizers, preservatives and antioxidants are usually incorporated.

For example, for use on the skin commercially prepared bases such as oil-in-water and water-in-oil emulsions having excellent skin tolerance can be used as a support of the formulations, different types of gels can also be used due to the product characteristics. To evaluate the effect of the olive leaf extract on skin wounds in humans, the invention uses a water-in-oil cream and gelled water at the concentrations used in the animals with extraordinarily favorable results in all the treated cases as can be seen in the following photographs.

### Brief Description of the Drawings

Figure 1A and B compare two optical microscopy images of mesenchymal stem cells. The cells treated with olive leaf extract, image B, form tubular structures typical of the angiogenic process. Those same cells without treatment with olive leaf extract are unable to form said structures, image A.
Figure 2 shows images obtained with an optical microscope, comparing the formation of tubular structures in HUVECs cells in endothelial medium without treatment (Figure 2A), treated with VEGF (Figure 2B) and treated with olive leaf extract (Figure 2C) on Matrigel.
Figure 3A shows the results of the treatments with the leaf extract and 3B shows the results of the treatments with pure oleuropein. The progress of the wounds that have directly received treatment is shown in the graphs on the right, while the progress of those which have not received treatment is shown in the graphs on the left.
Figure 4 shows the response in the wounds of an animal to which 10⁻⁷ M oleuropein was administered.
Figure 5 shows the response in the wounds of an animal to which the olive leaf extract containing 10⁻⁵ M oleuropein was administered.
Figure 6 shows the progress of the ulcer of a patient to whom the composition of the invention was applied from day 0 (6A) to day 11 (6B).

### Examples

The angiogenic activity of olive leaf extracts which at least comply with the olive leaf extract requirements published in Pharmeuropa Olive [(2007) Leaf dry extract Pharmeuropa 19(3): 510-511] is demonstrated by:
1) Phenotypic evidence: after 15-28 days of culture of mesenchymal stem cells with olive leaf extracts typical endothelial cell structures such as microvessels are formed.
2) Evidence of expression of membrane markers: Treatment with olive leaf extracts increases cells with surface markers CD144 and VEGFR2
3) Evidence of expression of genes involved in endothelial differentiation: VEGF, PCAM, PDGFR and VEGFR1

This evidence is demonstrated by means of the following examples.

### 1. Angiogenesis and vasculogenesis induction and stimulation assays.

### Mesenchymal stem cell culture

Mesenchymal stem cells obtained from human bone marrow were expanded and grown in alpha-modified minimum essential medium (α-MEM) supplemented with 10% fetal bovine serum, 2 mM ultraglutamine and with antibiotics. When the cells were near confluence they were treated with olive leaf extracts. As controls, part of the cultures were not treated.

The cells were incubated at 37°C with 5% CO₂ and the media were changed every 3 days throughout the experiment.

### Endothelial cells

The established line of human umbilical vein endothelial cells (HUVE) was supplied by Lonza Group, Ltd. Switzerland. These cells were expanded in a flask with endothelial basal medium (EBM, Lonza) supplemented with antibiotics and fetal bovine serum. The cells were subcultured when they reached confluence. The medium was changed every 3 days. Second and third pass cells were used to carry out these experiments. The treatments these cells received were:
1.- Leaf extract at different concentrations
2.- 10 µg/mL VEGF
3.- Control, without treatment

### Flow cytometry and monoclonal antibody studies

Flow cytometry assays were conducted for the study and analysis of membrane cell markers. The cells were washed with PBS + 3% fetal bovine serum, conjugated antihuman monoclonal antibodies CD144-FITC and VEGFR2-PE were incubated for 30 minutes at room temperature. The labeled cells were washed three times, resuspended in 0.5 ml of PBS and the labeling analyzed with a FACScan Caliber flow cytometer (Benton-Dickinson).

### Real-time polymerase chain reaction (RT-PCR)

Real-time PCR was used to analyze the expression of angiogenic gene markers. The total RNA of the cells was obtained using the reagent Tri Reagent (Sigma Aldrich) and following the manufacturer's instructions. The cDNA was synthesized from a microgram of total RNA which had previously been treated with DNAsal (Sigma Aldrich) using the iSCript TMcDNA Biorad Synthesis Kit. The real-time PCR used for quantifying the analyzed mRNA was carried out in a Light-Cycler system (Roche) using SYBR®Green. These reactions were performed in a final volume of 10 µL with 1 µL of cDNA, 10 pmol of each primer and Quantitec® SYBR®Green master mix (Qiagen). The conditions for the Light-Cycler were 95°C for 15 minutes, 40 cycles of 95°C for 30 seconds, 60°C for 15 seconds and 72°C for 30 seconds. The threshold cycle (Ct) of the target gene was standardized with the corresponding threshold cycle of the constitutive gene pGAPDH.

### Angiogenesis

The Endothelial Tube Formation Assay kit, Cell Biolab Inc., was used to carry out the *in vitro* angiogenesis assays. The assay conditions are those which were indicated in the supplier's instructions. The media used were supplemented endothelial basal medium untreated (negative control) and treated with VEGF (positive control) or with olive leaf extract. The structures formed were labeled by means of an antibody conjugated with calcein AM supplied in the kit specific for tubules.

### Results obtained in the differentiation of mesenchymal stem cells into endothelial cells

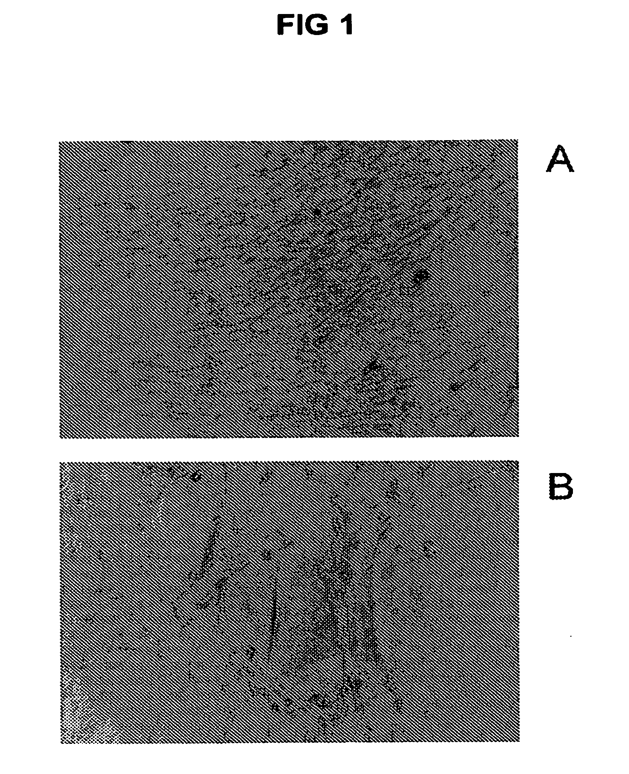

The capacity of olive leaf extracts to promote angiogenesis in stem cells is shown in Figure 1, where the tubular structures characteristic of endothelial cells obtained by differentiation of mesenchymal stem cells treated with the olive leaf extracts can be observed.

Cytometric analysis of the surface markers typical of endothelial cells, such as CD144 and VEGFR2, shows that they are found in MSCs treated with olive leaf extract and in all the analyzed cases the percentage of these endothelial markers in cells treated with the extracts show significant differences with respect to the control culture.

The gene expression of different genes involved in endothelial differentiation such as VEGF, PCAM, PDGFR and VEGFR1, shows an increase with treatment with olive leaf extract.

### Results obtained in the induction of angiogenesis in endothelial cells

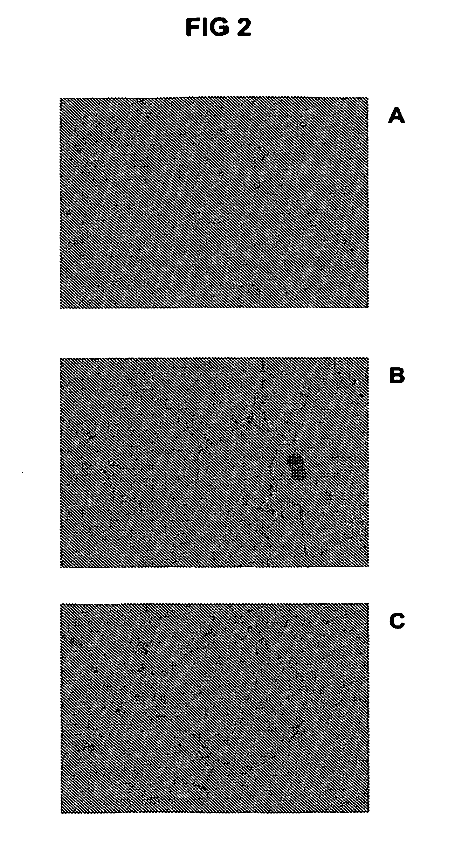

The capacity of olive leaf extracts to promote angiogenesis in endothelial cells was tested and evaluated by means of cell experiments in a Matrigel substrate. The endothelial cells treated with olive leaf extract had a capacity comparable to VEGF in promoting the formation of tubular structures typical of blood vessel formation (Figure 2).

### Comparison of the results with the state of the art

As previously stated in preceding sections herein, angiogenesis begins with tissue damage, which activates endothelial cell proliferation and the assembly thereof into tubular structures around which the new vessel walls are formed [Karamysheva AF. Mechanisms of angiogenesis 2008 Biochemistry]. The results herein presented with olive leaf extracts allow proposing the application of such extracts as an angiogenic factor due to their capacity of differentiating stem cells into endothelial cells capable of forming tubular structures.

The results obtained with olive leaf extracts as an angiogenesis promoter are comparable to or better than those of VEGF. This factor is one of the most important angiogenic agents and is involved in current studies having the objective of finding therapies for pathologies relating to angiogenesis, such as ischemias or wound healing, *inter alia.* In the presented results the effects shown by olive leaf extracts are similar to or better than those shown by VEGF, which supports the use of these extracts as angiogenic inducers [Jabbarzadeh, E. Induction of angiogenesis in tissue-engineered scaffolds designed for bone repair: a combined gene therapy-cell transplantation approach. PNAS 2008]; [Guerrero, M., K. Athota, et al. (2008). "Vascular endothelial growth factor-165 gene therapy promotes cardiomyogenesis in reperfused myocardial infarction." J Interv Cardiol 21 (3): 242-51]; [Michaels, J. T., M. Dobryansky, et al. (2005). "Topical vascular endothelial growth factor reverses delayed wound healing secondary to angiogenesis inhibitor administration." Wound Repair Regen 13(5): 506-12]; [Galiano, R. D., O. M. Tepper, et al. (2004). "Topical vascular endothelial growth factor accelerates diabetic wound healing through increased angiogenesis and by mobilizing and recruiting bone marrow-derived cells." Am J Pathol 164(6): 1935-47].

The treatment of human mesenchymal stem cells with olive leaf extracts has favored differentiation of these cells into endothelial cells, and more over into cells that have been capable of forming the tubular structures required in any angiogenic and vasculogenic process. This is consistent with that described by Alviano *et al., inter alia,* who have shown the differentiation potential of mesenchymal stem cells into endothelial cells under the influence of angiogenic factors such as VEGF [Alviano, F., V. Fossati, et al. (2007). "Term Amniotic membrane is a high throughput source for multipotent Mesenchymal Stem Cells with the ability to differentiate into endothelial cells in vitro." BMC Dev Biol 7: 11].

The results obtained both in the gene expression of endothelial markers and in the analysis of surface markers of mesenchymal stem cells differentiated into endothelial cells with leaf extracts and VEGF shows that the inductive effect of these extracts thereon is comparable to that of VEGF, a recognized inducer for differentiating these cells into endothelial cells.

### 2. Healing induction and stimulation assays

### 2.a) Assays in animals

Female db/db mice (BKS.Cg-m +/+ Lepr^{db}) aged 10 to 12 weeks were used. They were obtained through Charles River Laboratories (Spain), the average weight of the animals upon reception was 35.64 g. Throughout the entire study, the animals were kept in animal houses particularly designed for allowing control of the animals' environment, such as relative humidity (30-70%), temperature (22±2°C), air pressure, number of renewals and light period (12/12 hours of light/darkness). Access to water was *"ad libitum"* and the diet was a standard dry pellet diet for rats supplied by PanLab (Barcelona, Spain). After the acclimatization period, the mice were randomly distributed into the different study groups. In this study three different doses of an olive leaf extract were used, the oleuropein content of which was 41.5%; furthermore, pure oleuropein was used in other animal groups in the three concentrations used in the extract. rhVegf (Sigma Aldrich) was used as the reference substance and a placebo group was used as control of the experiment; all the compounds used in this study were dissolved in PBS. The concentrations used in this assay are detailed in Table 1.

**Table 1: Compositions administered to the different study groups and their respective concentrations**

| Group | Assay substance | Concentration |
|---|---|---|
| A | Placebo (PBS) | |
| B | Leaf extracts | 10e-2m oleuropein |
| C | Leaf extracts | 10e-5m oleuropein |
| D | Leaf extracts | 10e-7m oleuropein |
| E | Oleuropein | 10e-2m oleuropein |
| F | Oleuropein | 10e-5m oleuropein |
| G | Oleuropein | 10e-7m oleuropein |
| H | VEGF (reference) | 5 µg.ml⁻¹ |

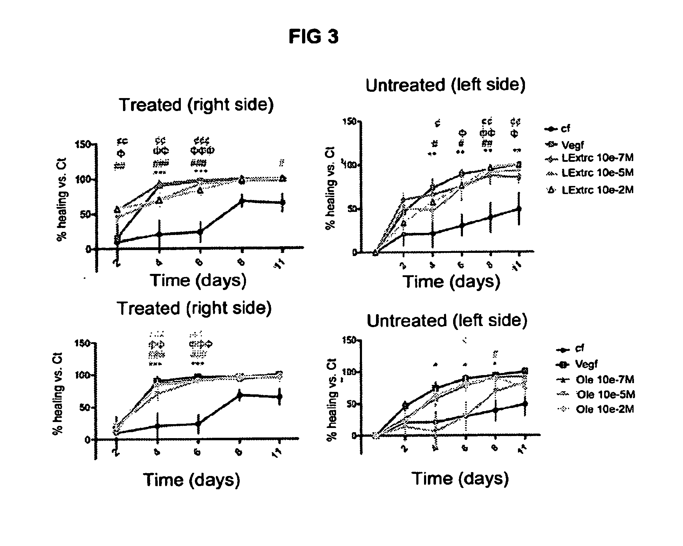

On day 0 of the experiment, two 6 mm excisional wounds were made on the back of every mouse under aseptic conditions and using surgical instruments. Every mouse received in one of the wounds (right side) the treatment corresponding to its group and received no treatment in the other (left side). The treatments were administered topically on the wounds on days 0, 2, 4, 6 and 8 of the experiment. The wound healing process was tracked and evaluated throughout the experiment (see Figure 3).

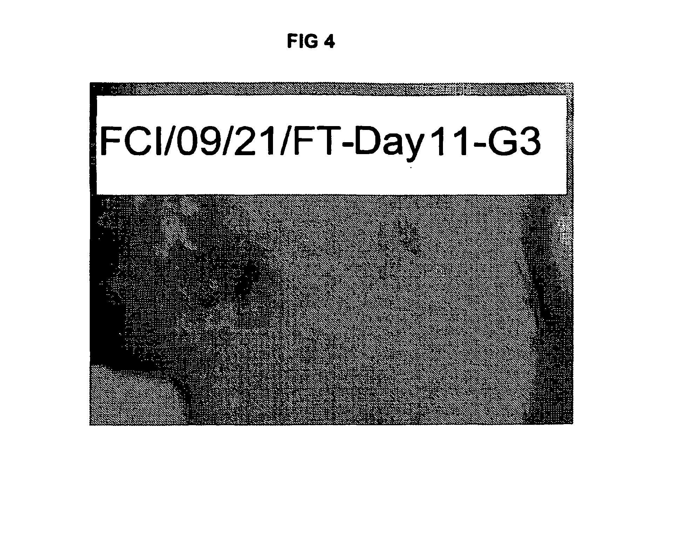

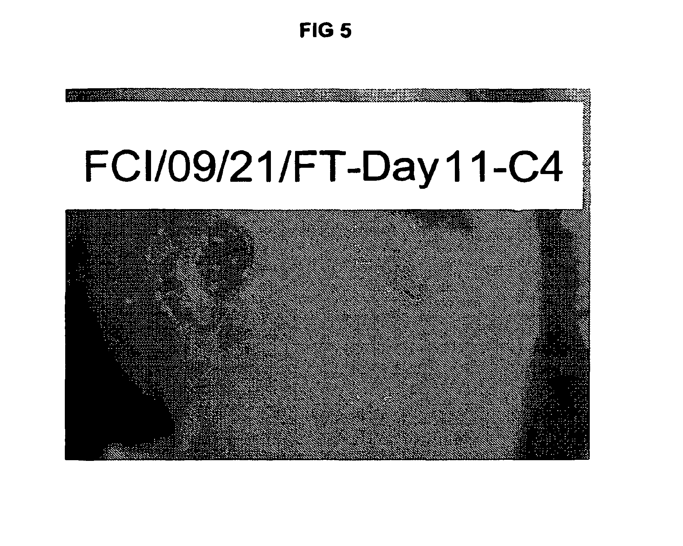

On the treatment administration days photographs were taken of both wounds in every mouse for subsequent evaluation (see Figures 4 and 5).

In order to track the wound healing, a digital planimetry method was used whereby the area of every wound was quantified. The inter-group differences have been determined by means of a two-way ANOVA statistical analysis followed by a Bonferroni post-hoc analysis. Differences have been considered when p<0.05

The results of these experiments show a very positive effect of olive leaf extracts in wound healing, similar to those observed when oleuropein is used. Both the wound that received treatment (right side) and the wound that did not (left side) show increased healing (Figure 3) which is statistically significant with respect to the control (group A-treatment with PBS in both wounds).

In all the evaluated times and with the three doses of leaf extracts analyzed a healing effect comparable to that produced by VEGF is observed in both wounds. Even in the case of directly treated wounds, it is observed that the difference with the control group starts sooner in time, after two days, with the three doses of leaf extracts used than with the VEGF.

### 2.b) Assays in humans

The formulation of the invention for applying to humans can be prepared in its simplest or most elementary form by dissolving the corresponding olive leaf extract containing a known concentration of oleuropein, in concentrations ranging from **10⁻²** M to 10⁻⁷ M, in a mixture with water gel or common commercial moisturizing creams, to give them consistency and facilitate the local application on the affected area of the skin. The mentioned formulations were evaluated with elderly patients and/or diabetic patients with ulcers of different etiological types. No patient showed hypersensitivity to the extracts and they showed a positive response that started to be seen on the first days of treatment, results being optimal before the month of treatment was reached in all cases.

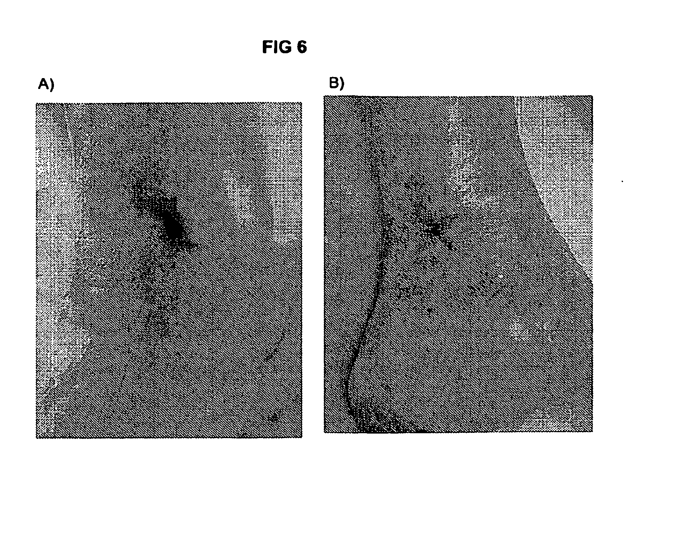

Concerning adverse effects, reddening was observed in only one case but the administration of the product did not have to be stopped in any case. Figure 6 shows the results obtained in one patient.

## Claims

1. Olive leaf extract for use in the treatment of skin wounds.

2. A pharmaceutical composition for use in the treatment of skin wounds comprising the olive leaf extract according to claim 1 adapted for topical administration.

3. A pharmaceutical composition for use according to claim 2 in the form of a cream, ointment, balsam, solution, emulsion, transdermal patch or salve.

4. A pharmaceutical composition for use according to any of claims 2 or 3, wherein the pharmaceutical composition comprises a concentration of less than 30% of the olive leaf extract of claim 1.

5. A pharmaceutical composition for use according to claim 4, wherein the pharmaceutical composition comprises from 1 - 10% of the olive leaf extract of claim 1.

6. A pharmaceutical composition for use according to any of claims 2 to 5, wherein the treatment comprises application of the pharmaceutical composition to the wound or ulcer from 1 to 8 times a day.

7. A pharmaceutical composition for use according to any of claims 2 to 6, wherein the treatment comprises application of the pharmaceutical composition to the wound for the first time up to 14 days after the onset of the damage.

8. A pharmaceutical composition for use according to any of claims 2 to 7 further comprising polyphenols and/or angiogenesis- and vasculogenesis-stimulating factors.

9. A pharmaceutical composition for use according to any of claims 2 to 8, further comprising mesenchymal stem cells.

## Patentansprüche

1. Olivenblattextrakt für dessen Verwendung bei der Behandlung von Hautwunden.

2. Pharmazeutische Zusammensetzung für deren Verwendung bei der Behandlung von Hautwunden, umfassend den Olivenblattextrakt nach Anspruch 1, welche für topische Verabreichung geeignet ist.

3. Pharmazeutische Zusammensetzung für deren Verwendung nach Anspruch 2 in Form einer Creme, eines Liniments, eines Balsams, einer Lösung, einer Emulsion, eines transdermalen Pflasters oder einer Salbe.

4. Pharmazeutische Zusammensetzung für deren Verwendung nach einem der Ansprüche 2 oder 3, wobei die pharmazeutische Zusammensetzung eine Konzentration von weniger als 30% des Olivenblattextraktes nach Anspruch 1 umfasst.

5. Pharmazeutische Zusammensetzung für deren Verwendung nach Anspruch 4, wobei die pharmazeutische Zusammensetzung von 1 - 10% des Olivenblattextraktes nach Anspruch 1 umfasst.

6. Pharmazeutische Zusammensetzung für deren Verwendung nach einem der Ansprüche 2 bis 5, wobei die Behandlung die Anwendung der pharmazeutischen Zusammensetzung auf der Wunde oder dem Geschwür von 1 bis 8 Mal am Tag umfasst.

7. Pharmazeutische Zusammensetzung für deren Verwendung nach einem der Ansprüche 2 bis 6, wobei die Behandlung die Anwendung der pharmazeutischen Zusammensetzung auf der Wunde zum ersten Mal bis 14 Tagen nach dem Ausbruch der Verletzung umfasst.

8. Pharmazeutische Zusammensetzung für deren Verwendung nach einem der Ansprüche 2 bis 7, zusätzlich umfassend Polyphenole und/oder Angiogenese- und Vaskulogenesestimulierende Faktoren.

9. Pharmazeutische Zusammensetzung für deren Verwendung nach einem der Ansprüche 2 bis 8, zusätzlich umfassend mesenchymale Stammzellen.

## Revendications

1. Extrait de feuilles d'olivier pour son utilisation dans le traitement de blessures cutanées.

2. Composition pharmaceutique pour son utilisation dans le traitement de blessures cutanées comprenant l'extrait de feuilles d'olivier selon la revendication 1 adaptée pour l'administration topique.

3. Composition pharmaceutique pour son utilisation selon la revendication 2 sous la forme d'une crème, une pommade, un baume, une solution, une émulsion, un timbre transdermique ou un onguent.

4. Composition pharmaceutique pour son utilisation selon l'une quelconque des revendications 2 ou 3, dans laquelle la composition pharmaceutique comprend une concentration inférieure à 30% de l'extrait de feuilles d'olivier selon la revendication 1.

5. Composition pharmaceutique pour son utilisation selon la revendication 4, dans laquelle la composition pharmaceutique comprend 1 - 10% de l'extrait de feuilles d'olivier selon la revendication 1.

6. Composition pharmaceutique pour son utilisation selon l'une quelconque des revendications 2 à 5, dans laquelle le traitement comprend l'application de la composition pharmaceutique à la blessure ou ulcère 1 à 8 fois par jour.

7. Composition pharmaceutique pour son utilisation selon l'une quelconque des revendications 2 à 6, dans laquelle le traitement comprend l'application de la composition pharmaceutique à la blessure pour la première fois jusqu'à 14 jours après le début des dommages.

8. Composition pharmaceutique pour son utilisation selon l'une quelconque des revendications 2 à 7, comprenant en outre des polyphénols et/ou des facteurs de stimulation de l'angiogenèse et de la vasculogenèse.

9. Composition pharmaceutique pour son utilisation selon l'une quelconque des revendications 2 à 8, comprenant en outre des cellules souches mésenchymateuses.
